(19) Europäisches Patentamt
European Patent Office
Office européen des brevets

(11) **EP 2 309 956 B1**

(12) **EUROPÄISCHE PATENTSCHRIFT**

(45) Veröffentlichungstag und Bekanntmachung des Hinweises auf die Patenterteilung:
**07.09.2016 Patentblatt 2016/36**

(21) Anmeldenummer: **09772151.8**

(22) Anmeldetag: **01.07.2009**

(51) Int Cl.:
**A61F 13/00** *(2006.01)*

(86) Internationale Anmeldenummer:
**PCT/EP2009/004734**

(87) Internationale Veröffentlichungsnummer:
**WO 2010/000451 (07.01.2010 Gazette 2010/01)**

(54) **WUNDAUFLAGE UND VERFAHREN ZU IHRER HERSTELLUNG**

WOUND DRESSING AND METHOD FOR ITS MANUFACTURE

PANSEMENT ET SON PROCÉDÉ DE FABRICATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **03.07.2008 DE 102008031183**

(43) Veröffentlichungstag der Anmeldung:
**20.04.2011 Patentblatt 2011/16**

(73) Patentinhaber: **Paul Hartmann AG**
**89522 Heidenheim (DE)**

(72) Erfinder: **JUNGINGER, Martin**
**89568 Hermaringen (DE)**

(56) Entgegenhaltungen:
**EP-A1- 0 691 113     GB-A- 2 428 581**
**US-A- 5 292 777**

**Beschreibung**

Die Erfindung betrifft Wundauflagen insbesondere als Wundbehandlungsmittel in der Granulations- und Epitelisierungsphase. Diese Wundauflagen können insbesondere zur feuchten Wundbehandlung eingesetzt werden. Darüber hinaus betrifft die Erfindung ein Verfahren zur Herstellung dieser Wundauflagen.

Die Heilung von Hautwunden beruht auf der Fähigkeit der Haut Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).

Zur Unterstützung der einzelnen Wundheilungsphasen sind zahlreiche Vorschläge in der Literatur beschrieben. Insbesondere sind auch Wundauflagen mit Polyurethanschäumen seit geraumer Zeit Gegenstand zahlreicher Artikel in der Fachliteratur als auch von Patentliteratur. So werden beispielsweise mit den europäischen Patenten EP 457 977 B1, EP 486 522 B1, EP 541 390 B1, EP 541 391 B1, EP 570 430 B1, EP 665 856 B1, EP 691 113 B1, EP 693 913 B1 oder EP 1 082 146 B1 Wundauflagen mit verschiedenen Konstruktionen beschrieben, die als absorbierende Schicht einen Polyurethanschaum umfassen. Diese Polyurethanschäume neigen unter Umständen dazu mit der heilenden Wunde zu verkleben, indem neue Zellen/ Gewebe in die Poren der Schäume einwachsen.

Darüber hinaus sind mit den europäischen Patenten EP 855 921 B1 und EP 1 156 838 B1 Wundauflagen bekannt, die einen Polyurethanschaum umfassen, der mit einem hydrophoben Silikongel beschichtet ist. Dieses Silikongel soll die Verklebung der Wunde mit dem Polyurethanschaum verhindern.

Die GB 2 428 581 A beschreibt eine Wundauflage umfassend eine Lage aus einem hydrophilen Schlaumstoffmaterial, das mit einer Wärme erzeugenden Zusammensetzung beschichtet ist.

Weiterhin werden mit den internationalen Anmeldungen WO 02/ 38 097 A1, WO 02/45761 A1. WO 03/011 352 A1, WO 03/ 086 255 A1, WO 2004/ 052 415 A1 oder EP 1 658 865 A1 Wundauflagen beschrieben, die ein Hydrogel und einen Polyurethanschaum umfassen. Die verwendeten Polyurethanschäume sind trocken.

US 2004/0153040 A1 offenbart eine mehrschichtige Wundauflage mit einer Schicht aus Polyurethanschaum. Die Wundauflage kann vor Gebrauch mit Wasser beaufschlagt werden.

Ausgehend vom diesen beschriebenen und dem Markt zur Verfügung stehenden Wundauflagen ist es eine Aufgabe der vorliegenden Erfindung, eine verbesserte Wundauflage bereitzustellen, die insbesondere zur Wundheilung in der Granulations- und/ oder Epitelisierungsphase eingesetzt werden kann. Darüber hinaus soll eine Wundauflage bereitgestellt werden, die den pathologischen Zustand einer Wunde derart beeinflusst, dass ein normaler, natürlicher Wundheilungsverlauf stattfinden kann. Hierfür soll die Wundauflage eine ausreichende Menge an Feuchtigkeit der Wunde bereitstellen und gleichzeitig ein gutes Absorptionsvermögen aufweisen. Des Weiteren soll eine Wundauflage zur Verfügung gestellt werden, die möglichst geringe Scherkräfte auf eine zu behandelnde Wunde ausübt. Zur Lösung dieser Aufgaben wird eine mehrschichtige Wundauflage gemäß Anspruch 1 vorgeschlagen.

Insbesondere umfasst die zweite Schicht einer erfindungsgemäßen Wundauflage einen hydrophilen Polyurethanschaum, der einen Wasseranteil von mindestens 20 Gew.-% Wasser, insbesondere mindestens 30 Gew.-% und ganz besonders bevorzugt mindestens 35 Gew.-% Wasser umfasst. Der Polyurethanschaum weist einen Wasseranteil von höchstens 80 Gew.-% Wasser auf. Insbesondere ist der Wasseranteil höchstens 70 Gew.-% und ganz besonders bevorzugt höchstens 65 Gew.-%. Insbesondere ist dieser Wasseranteil homogen in der Polyurethanmatrix des Schaums verteilt. Insbesondere umfasst der hydrophile Polyurethanschaum dabei einen Wasseranteil von mindestens 10 Gew.-% und höchstens 80 Gew.-% Wasser, wobei das Wasser insbesondere homogen in dem Polyurethanschaum verteilt ist.

Hierbei und im Folgenden soll im Zusammenhang mit der vorliegenden Erfindung - falls nichts anderes angegeben ist - jeder Gehalt eines Inhaltstoffes in Gewichtsprozent (Gew.-%) bezogen auf das Gewicht der den Inhaltsstoff umfassenden Komponente verstanden sein.

Zur Überprüfung der Menge an Wasser in der jeweiligen Komponente soll im Zusammenhang mit der vorliegenden Erfindung die Norm DIN EN 14079 herangezogen werden, wobei die Menge an Wasser wie folgt berechnet wird:

$$W_w = \frac{W_g - W_t}{W_g} \bullet 100\% \qquad\qquad (1)$$

mit $W_w$ = Gewicht des Wassers in % bezogen auf das Gesamtgewicht der Komponente
$W_g$ = Gewicht der Wasser enthaltenden Komponente
$W_t$ = Gewicht der trockenen Komponente

Damit soll im Zusammenhang mit der vorliegenden Erfindung unter einem Polyurethanschaum mit einem Wasseranteil von mindestens 10 Gew.-% ein solcher Polyurethanschaum verstanden sein, der mindestens 10 Gew.-% Wasser umfasst, wobei das Wasser von dem Polyurethanschaum freigesetzt werden kann. Im Unterschied hierzu ist nicht der Anteil an Wasser zu verstehen, der eventuell zur Bildung beispielsweise bei der Polymerisation der Ausgangsprodukte des Polyurethanschaums verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des Polyurethanschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen Polyurethanschaum auf, der einen Wasseranteil umfasst, der deutlich einen eventuell durch die Herstellung nach Trocknung bedingten Restgehalt an Wasser übersteigt.

Weiterhin bevorzugt umfasst eine erfindungsgemäße Wundauflage einen hydrophilen Polyurethanschaum, der einen Retentionswert R von mindestens 20 % aufweist. Hierbei ist weiterhin bevorzugt vorgesehen, dass der hydrophile Polyurethanschaum einen Retentionswert R von mindestens 30 %, insbesondere von mindestens 40 %, insbesondere mindestens 40 % und ganz besonders bevorzugt von mindestens 50 % aufweist. Unabhängig hiervon kann weiterhin bevorzugt vorgesehen sein, dass die Wundauflage einen hydrophilen Polyurethanschaum aufweist, der einen Retentionswert R von höchstens 90 %, insbesondere von höchstens 80 % und ganz besonders von höchstens 70 % aufweist. Der Retentionswert R wird dabei gemäß einer hierin beschriebenen Methode bestimmt.

Ganz besonders bevorzugt umfasst eine erfindungsgemäße Wundauflage einen hydrophilen Polyurethanschaum, der einen Wasseranteil von mindestens 10 Gew.-% umfasst, wobei der Wasseranteil dem Retentionswert R des Polyurethanschaums entspricht.

Somit kann eine Polyurethanschaum-Wundauflage bereitgestellt werden, die bestens für die moderne Wundbehandlung konditioniert ist, da die Wundauflage durch den Wassergehalt in dem Polyurethanschaum einer zu behandelnden Wundoberfläche sofort nach der Applikation ein besonders wundheilungsförderndes Klima zur Verfügung stellt. Die Wunde wird von Anfang an mit Feuchtigkeit oder Wasser versorgt, wobei die Wundauflage gleichzeitig durch den absorbierenden Polyurethanschaum eine ausreichende Absorptionskapazität aufweist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt. Aufgrund der etwas geringeren Absorptionskapazität gegenüber trockenen Polyurethanschäumen, die kein freisetzbares Wasser umfassen und meist sehr schnell sehr viel Wundsekret aufnehmen, wodurch eine trockene Wundoberfläche gebildet wird, ist diese Wundauflage hervorragend geeignet in der Epitelisierungs- oder Granulationsphase der Wundheilung eingesetzt zu werden. Damit kann die erfindungsgemäße Wundauflage in natürlicher Weise die Granulation und/ oder die Epiteliesierung der Wunde in besonderem Maße fördern.

Darüber hinaus kann eine Wundauflage bereitgestellt werden, die gegenüber Wundauflagen mit trockenen hydrophilen Polyurethanschäumen eine sehr viel geringere Scherbelastung auf die Wunde ausübt. Durch einen Wasseranteil von mindestens 10 Gew-% Wasser in dem Polymerschaum kann eine Wundauflage mit einem vorkonditionierter hydrophilen Polyurethanschaum bereitgestellt werden, die bei Absorption von Flüssigkeiten gegenüber einer Wundauflage mit einem trockenen hydrophilen Polyurethanschaum ein sehr viel geringeres Quellvermögen aufweist. Durch das geringere Quellvermögen des vorkonditionierten Polyurethanschaums herrschen somit innerhalb der Wundauflage geringere Scherkräfte in Bezug auf weitere Lagen oder Materialien als auch in Bezug auf eine zu behandelnde Wunde. Hierdurch kann eine Polyurethanschaum-Wundauflage bereitgestellt werden, die besonders wundheilungsfördernd ist.

Damit ist gemäß einem weiterführenden Gedanken auch eine Wundauflage Gegenstand der Erfindung, die eine zweite Lage mit einer ersten Seite und einer zweiten Seite umfasst, wobei die Lage einen hydrophilen Polyurethanschaum umfasst, der einen Wasseranteil von mindestens 10 Gew.-% umfasst und der ein Quellvermögen $\Delta V_1$ von höchstens 80 % aufweist. Insbesondere weist der hydrophile Polyurethanschaum dabei ein Quellvermögen $\Delta V_1$ von höchstens 60 %, insbesondere von höchstens 40 %, insbesondere von höchstens 30 % und ganz besonders bevorzugt von höchstens 20 % auf. Dabei kann weiterhin vorteilhaft vorgesehen sein, dass der Polyurethanschaum ein Restquellvermögen $\Delta V_1$ von mindestens 5 % aufweist. Dieses Restquellvermögen kann ausgenutzt werden, damit die Wundauflage während der Absorption einen besseren Kontakt zum Wundgrund annehmen kann.

Hierbei soll unter dem Quellvermögen $\Delta V_1$ eines Polyurethanschaums die Volumenzunahme verstanden sein, die ein Polyurethanschaum, der seine Absorptionskapazität vollständig erschöpft hat, im Vergleich zu einem Polymerschaum mit einem Wassergehalt von mindestens 10 Gew.-% Wasser erfährt. Das Quellvermögen soll dabei gemäß einem hierin beschrieben Test ermittelt werden.

Als Polyurethanschaum kann im Zusammenhang mit der vorliegenden Erfindung jeder heute in der modernen Wundbehandlung übliche hydrophile Polyurethanschaum verwendet werden, der einen Wasseranteil in seine Polyurethanmatrix aufnimmt und eine ausreichende Absorption aufweist. Damit ist im Zusammenhang mit der vorliegenden Erfindung unter einem hydrophilen Polyurethanschaum ein solcher Polyurethanschaum zu verstehen, der eine Flüssigkeit in seine Polyurethanmatrix und in seine Poren aufnehmen und speichern, somit absorbieren kann, und zumindest einen Teil der aufgenommene Flüssigkeit wieder abgeben kann. Als hydrophile Polymerschäume sind hierbei insbesondere offenporige, hydrophile Polyurethanschäume geeignet. Dem gemäß umfasst eine besonders bevorzugte Wundauflage eine zweite Schicht, die einen offenporigen, hydrophilen Polyurethanschaum umfasst.

Erfindungsgemäß sollen solche Polyurethanschäume eingesetzt werden, die eine hohe Absorptionskapazität aufweisen. Diese Absorptionskapazität soll vorhanden sein, obwohl der Polyurethanschaum einen Anteil seines Eigengewichtes an Wasser in seine Polymermatrix aufgenommenen hat. Gemäß der Erfindung umfasst damit eine erfindungsgemäße Wundauflage einen Polyurethanschaum, der einen Wasseranteil von mindestens 10 Gew.-% und höchstens 80 Gew.% Wasser umfasst und der eine freie Absorption $A_2$ von mindestens 10 g/ g aufweist. Insbesondere weist die Wundauflage eine freie Absorption $A_2$ von mindestens 12 g/ g und ganz besonders bevorzugt von mindestens 15 g/ g auf, wobei die freie Absorption $A_2$ gemäß der DIN-EN 13726-1 (2002) bestimmt wird. Hierbei ist die freie Absorption $A_2$ die freie Absorption des Wasser enthaltenden Polyurethanschaums.

Weiterhin bevorzugt umfasst eine erfindungsgemäße Wundauflage einen Polyurethanschaum, der einen Wasseranteil von mindestens 10 Gew.-% und höchstens 80 Gew.-% Wasser umfasst und der eine freie Absorption $A_1$ von mindestens 10 g/ g, insbesondere mindestens 12 g/ g und ganz besonders bevorzugt von mindestens 15 g/ g aufweist, wobei die freie Absorption $A_1$ gemäß der DIN-EN 13726-1 (2002) bestimmt wird. Hierbei ist die freie Absorption $A_1$ die freie Absorption des trockenen Polyurethanschaums.

Weiterhin bevorzugt umfasst eine erfindungsgemäße Wundauflage einen hydrophilen Polyurethanschaum, der eine durchschnittliche Porengröße von weniger als 1000 $\mu$m, insbesondere 100 bis 1000 $\mu$m, insbesondere 100 bis 500 $\mu$m und ganz besonders bevorzugt 100 bis 300 $\mu$m aufweist. Hierbei kann insbesondere vorgesehen sein, dass die durchschnittliche Porengröße an der ersten Seite der zweiten Schicht gleich groß ist wie die Porengröße im inneren der zweiten Schicht und/ oder gleich groß ist wie an der zweiten Seite der zweiten Schicht. Weiterhin bevorzugte hydrophile Polyurethanschäume weisen eine Dichte von weniger als 150 kg/ m$^3$, insbesondere weniger als 140 kg/ m$^3$ und ganz besonders bevorzugt 50 bis 120 kg/ m$^3$ auf.

Als besonders vorteilhafte Ausführungen haben sich weiterhin Wundauflagen gezeigt, die einen Polyurethanschaum umfassen, dessen Schichtdicke 0,1 bis 5,0 mm aufweist. Insbesondere weist damit eine erfindungsgemäße Wundauflage eine absorbierende Schicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen einerseits die Fähigkeit ein von einer Wunde abgegebenes Wundexsudat aufzunehmen zu können und gleichzeitig eine ausreichende Menge an Wasser oder Feuchtigkeit einer Wund bereitstellen zu können. Diese Schichtdicken können an jeder Stelle der Wundkontaktschicht gleich sein oder in verschiedenen Bereichen der Wundkontaktschicht verschiedene Werte annehmen. Insbesondere ist auch vorgesehen, dass die absorbierende Schicht oder der Polyurethanschaum abgeflachte Ränder aufweist.

Als Wundkontaktschicht kann gemäß der vorliegenden Erfindung ein Material Verwendung finden, das keinen negativen Einfluss auf die Wundheilung ausübt. Hierbei steht eine Wundkontaktschicht bei Verwendung der erfindungsgemäßen Wundauflage in direktem Kontakt mit der Wunde. Diese Wundkontaktschicht kann einzig und allein dazu dienen, dass der Polyurethanschaum von der zu behandelnden Wunde beabstandet wird, als auch dass diese Wundkontaktschicht weitere Funktionen in Bezug auf die Wundauflage als auch auf die zu behandelnde Wunde ausübt. Insbesondere kann eine erfindungsgemäße Wundauflage eine Wundkontaktschicht mit einer ersten Seite und einer zweiten Seite umfassen, wobei die Wundkontaktschicht eine Hydrogelmatrix, einen Polymerfilm, eine Hydrokolloidmatrix, ein Polymernetz, ein Nonwoven oder ein Adhäsiv umfasst.

Gemäß einer Weiterbildung der erfindungsgemäßen Wundauflage kann vorgesehen sein, dass die Wundkontaktschicht eine Vielzahl an Kanälen, Öffnungen oder Löcher zum Durchtritt von Flüssigkeiten umfasst. Insbesondere ist dabei vorgesehen, das die Wundkontaktschicht Kanäle aufweist, die einen Durchtritt von Wundsekret von der ersten Seite zur zweiten Seite bilden. In dieser Ausführungsform ist vorgesehen, dass die erste Seite der Wundkontaktschicht einen direkten Kontakt zu einer zu behandelnden Wunde und die zweite Seite der Wundkontaktschicht einen direkten Kontakt mit der ersten Seite der absorbierenden Schicht aufweist.

Gemäß einer Weiterbildung der Erfindung kann auch vorgesehen sein, dass die Wundkontaktschicht Kanäle, Öffnungen oder Löcher aufweist, die einen Durchmesser von 0,5 bis 5 mm aufweisen. Insbesondere weist die Wundkontaktschicht Kanäle, Öffnungen oder Löcher auf, die einen Durchmesser von 1 bis 3 mm aufweisen. Ganz besonders bevorzugt weist die Wundkontaktschicht auf der im anwendungsgerechten Zustand der Wundauflage der Wunde zugewandten, ersten Seite Öffnungen auf, die einen Durchmesser von 1 bis 3 mm aufweisen, wobei die zweite Seite der Wundkontaktschicht in direktem Kontakt mit dem Polyurethanschaum steht.

Weiterhin bevorzugt kann die Wundkontaktschicht eine Vielzahl an Kanälen, Öffnungen oder Löcher zum Durchtritt von Flüssigkeiten aufweisen, wobei die Kanäle, Öffnungen oder Löcher an der ersten Seite der Wundkontaktschicht eine Fläche von höchstens 95 % der Fläche der ersten Seite der Wundkontaktschicht einnehmen. Hierbei ist weiterhin bevorzugt vorgesehen, dass die Kanäle, Öffnungen oder Löcher eine Fläche von höchstens 70 %, insbesondere höchstens 50 %, insbesondere höchstens 40 % und ganz besonders bevorzugt von höchstens 30 % der Fläche der ersten Seite der Wundkontaktschicht einnehmen. Ganz besonders bevorzugt weist die Wundkontaktschicht Kanäle, Öffnungen oder Löcher auf, die an der ersten Seite der Schicht eine Fläche von mindestens 5 % und höchstens 30 % der Fläche der ersten Seite der Wundkontaktschicht einnehmen. Ganz besonders weist die Wundauflage eine Wundkontaktschicht auf, die 2 bis 8 Löcher pro cm$^2$ aufweist. Hierbei kann vorgesehen sein, dass die Wundkontaktschicht ein gelochter Polymerfilm oder ein Polymernetz, insbesondere ein Polyurethanfilm oder ein Polymernetz ist.

Gemäß einer weiteren Ausführungsform der vorliegenden Erfindung kann weiterhin vorgesehen sein, dass eine erfindungsgemäße Wundauflage als Wundkontaktschicht eine Hydrokolloidmatrix umfasst. Diese Hydrokolloidmatrix kann aus einer klebenden Polymermatrix bestehen, in die Hydrokolloidpartikel dispergiert sind. Gemäß der vorliegenden Erfindung soll unter einem Hydrokolloid ein Material verstanden sein, das ein hydrophiles synthetisches oder natürliches Polymermaterial ist, das löslich oder absorbierend und/ oder quellend in Wasser ist. Vorzugsweise umfasst eine Wundkontaktschicht ein Hydrokolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum, Gelatine oder Mischungen hiervon.

Das Hydrokolloid kann sowohl in Form von Fasern als auch in Form von Partikeln und/ oder Fasern innerhalb einer Matrix vorliegen. Insbesondere kann das Hydrokolloid in Form von Partikeln in einer klebenden Polymermatrix vorliegen. Die klebende Polymermatrix umfasst dabei mindestens ein Co-Blockpolymer ausgewählt aus der Gruppe der AB-Diblock-Copolymere und/ oder ABA-Triblock-Copolymere, das aus den Monomeren Styrol, Butadien und Isopren aufgebaut ist. Der Anteil an Hydrokolloidpartikel in der Wundkontaktschicht kann vorzugsweise 10 bis 70 Gew.-% bezogen auf das Gesamtgewicht der Wundkontaktschicht aufweisen. Eine solche Zusammensetzung ist zum Beispiel mit der EP 1 007 597 B1 bekannt.

Gemäß einer weiteren Ausführungsform der vorliegenden Erfindung kann weiterhin vorgesehen sein, dass eine erfindungsgemäße Wundauflage als Wundkontaktschicht eine wasserhaltige Hydrogelmatrix umfasst. Diese Hydrogelmatrix kann vorzugsweise mindestens 20 Gew.-%, insbesondere mindestens 30 Gew.-%, insbesondere mindestens 40 Gew.-% und ganz besonders bevorzugt mindestens 50 Gew.-% Wasser umfassen, wobei die Hydrogelmatrix weiterhin bevorzugt höchstens 90 Gew.-% und weiterhin bevorzugt höchstens 80 Gew.-% Wasser umfasst. Somit kann eine Wundauflage bereitgestellt werden, die eine Wundkontraktschicht aufweist, die neben dem wasserhaltigen Polyurethanschaum eine zweite wasserhaltige Komponente enthält und somit eine für natürliche Wundheilung ausrechende Menge Feuchtigkeit zur Verfügung stellt.

Als wasserhaltige Hydrogelmatrices können im Zusammenhang mit der vorliegenden Erfindung insbesondere Hydrogelmatrices verwendet werden, die eine zusammenhängende, diskrete Schicht bilden und unter Druck kein Wasser abgeben. Insbesondere sind im Zusammenhang mit der vorliegenden Erfindung Hydrogelmatrices geeignet, die ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind besonders gut geeignet Wasser einzulagern und dieses Wasser an eine Wunde abzugeben.

Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt werden aus der Gruppe der Glykole, insbesondere Ethylenglykol oder Propylenglykol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar.
Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.-% eines mehrwertigen Alkohols und ganz besonders bevorzugt 10 bis 30 Gew.-% eines mehrwertigen Alkohols.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Ganz besonders bevorzugt umfasst die Hydrogelmatrix Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung.

Hierbei kann vorgesehen sein, dass die Hydrogelmatrix 0 bis 5 Gew.-% mindestens ein Salz umfasst. Insbesondere umfasst die Hydrogelmatrix 0,1 bis 3 Gew.-% eines Salzes und ganz besonders bevorzugt 0,5 bis 1,5 Gew.-% eines Salzes.

Insgesamt kann gemäß der vorliegenden Erfindung vorgesehen sein, dass die wasserhaltige Hydrogelmatrix bevorzugt mindestens 20 Gew.-% Wasser und mindestens 10 Gew.-% Polyurethan-Polyharnstoff-Copolymer umfasst. Eine weiterhin bevorzugte Hydrogelmatrix umfasst mindestens 20 Gew.-% Wasser und mindestens 15 Gew.-% Polyurethan-Polyharnstoff-Copolymer. Hierbei kann weiterhin bevorzugt vorgesehen sein, dass die Hydrogelmatrix aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols, 0 bis 5 Gew.-% mindestens eines Salzes ausgewählt aus der Gruppe Natriumchlorid, Kaliumchlorid, Magnesiumchlorid, Kalziumchlorid oder Mischungen hiervon und mindestens 20 Gew.-% Wasser gebildet wird.

Weiterhin bevorzugt kann vorgesehen sein, dass die Hydrogelmatrix aus 6 bis 30 Gew.-% eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols ausgewählt aus der Gruppe bestehend aus Propylenglycol und/ oder Glycerin, 0,5 - 1,5 Gew.-% eines Salzes ausgewählt aus der Gruppe Natriumchlorid, Kaliumchlorid, Magnesiumchlorid, Kalziumchlorid oder Mischungen hiervon und mindestens 30 Gew.-% Wasser gebildet wird.

Ganz besonders bevorzugt kann vorgesehen sein, dass die Hydrogelmatrix aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/ oder Glycerin, 0,5 bis 1,5 Gew.-% eines Salzes ausgewählt aus der Gruppe Natriumchlorid, Kaliumchlorid, Magnesiumchlorid, Kalziumchlorid oder Mischungen hiervon und mindestens 40 Gew.-% Wasser gebildet wird. Diese Hydrogelmatrix weist eine freie Absorption $A_3$ (gemessen nach DIN EN 13723-1 (2002)) von mindestens 1 g/ g und höchstens 5 g/ g auf, stellt ein nicht reizendes, flüssigkeitsabsorbierendes, vor Bakterien schützendes, polsterndes, hautartiges Medium bereit und ist somit besonders gut als Wundkontaktschicht geeignet.

Als besonders vorteilhafte Ausführungen haben sich weiterhin Wundauflagen gezeigt, die eine Hydrogelmatrix umfassen, deren Schichtdicke 0,1 bis 5,0 mm aufweist. Insbesondere weist damit eine erfindungsgemäße Wundauflage eine Wundkontaktschicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu der auf der zweiten Seite befindliche Öffnung größer ist.

Gemäß einer Weiterbildung der Erfindung kann auch vorgesehen sein, dass die Wundkontaktschicht oder die Hydrogelmatrix Öffnungen aufweist, die einen Durchmesser von 0,5 bis 5 mm aufweisen. Insbesondere weist die Wundkontaktschicht oder die Hydrogelmatrix Öffnungen auf, die einen Durchmesser von 1 bis 3 mm aufweisen. Ganz besonders bevorzugt weist die Wundkontaktschicht oder die Hydrogelmatrix auf der wundzugewandten ersten Seite Öffnungen auf, die einen Durchmesser von 1 bis 3 mm aufweisen, wobei die zweite Seite der Wundkontaktschicht oder der Hydrogelmatrix in direktem Kontakt mit dem Polyurethanschaum steht.

Es kann jedoch auch vorgesehen sein, dass zwischen der absorbierenden Schicht und der Wundkontaktschicht eine Übergangsschicht angeordnet ist. In dieser Ausführungsform weist eine erfindungsgemäße Wundauflage zwischen der Hydrogelmatrix und Polyurethanschaum eine Schicht auf, die beide Materialen umfasst. Diese Übergangsschicht kann ebenso wie die Wundkontaktschicht Kanäle, Öffnungen oder Löcher aufweisen. Falls die Übergangsschicht Kanäle, Öffnungen oder Löcher aufweist, sind gemäß einer weiter bevorzugten Ausführungsform diese Kanäle, Öffnungen oder Löcher mit Polyurethanschaum ausgefüllt. Weiterhin bevorzugt sind diese Kanäle, Öffnungen oder Löcher kongruent zu den Kanälen, Öffnungen oder Löchern der Wundkontaktschicht. Durch die Anordnung einer solchen Übergangsschicht kann eine Wundauflage bereitgestellt werden, die ein Laminat aus einem Polyurethanschaum und einer Hydrogelmatrix umfasst, das einen besonders festen Zusammenhalt zwischen der absorbierenden Schicht und der Wundkontaktschicht aufweist.

Gemäß einem weiteren weiterführenden Gedanken der vorliegenden Erfindung, ist auch eine Wundauflage Gegenstand der vorliegenden Erfindung, die zwischen der Hydrogelmatrix und dem hydrophilen Polymerschaum eine Barriereschicht aufweist. Eine solche Barriereschicht kann beispielsweise einen Polymerfilm umfassen, der mit Öffnungen versehen ist.

Weiterhin kann eine erfindungsgemäße Wundauflage eine Trägerschicht umfassen. Diese Trägerschicht kann aus verschiedenen Materialien bestehen. Üblicherweise werden in Wundauflagen textile Trägermaterialien, Nonwoven, Polymerfilme oder Polymerschäume eingesetzt. Diese Trägerschicht kann direkten Kontakt oder indirekten Kontakt zu der zweiten Seite der absorbierenden Schicht oder dem hydrophilen Polymerschaums aufweisen. Bei einem direkten Kontakt wird die Trägerschicht direkt auf die absorbierende Lage oder den Polyurethanschaum auflaminiert, wogegen bei einem indirekten Kontakt die Trägerschicht mittels eines Adhäsivs auf die absorbierende Schicht oder dem Polyurethanschaum aufgebracht ist. Dabei kann das Adhäsiv vollflächig oder lediglich in Teilbereichen zwischen der Trägerschicht und der absorbierenden Schicht aufgebracht sein.

Als Trägerschicht einer erfindungsgemäßen Wundauflage können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässigen und wasserdampfdurchlässigen Polyurethanfilm oder einen wasserundurchlässigen und wasserdampfdurchlässigen Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 $\mu$m, insbesondere 20 bis 40 $\mu$m und ganz besonders bevorzugt von 25 bis 30 $\mu$m aufweisen. Die Wasserdampfdurchlässigkeit des Polymerfilms der Wundauflage weist vorzugsweise mindestens 750 g/ m2/ 24 Std., insbesondere mindestens 1000 g/ m2/ 24 Std. und ganz besonders bevorzugt mindestens 2000 g/ m2/ 24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m2 zusammen mit dem Film eine Wasserdampfdurchlässigkeit von mindestens 800 g/ m2/ 24 Std. und vorzugsweise von mindestens 1000 g/ m2/ 24 Std. (gemessen nach DIN EN 13726) aufweisen.

Gemäß einem weiterführenden Gedanken der vorliegenden Erfindung, ist auch eine mehrschichtige Wundauflage umfassend eine Trägerschicht, eine absorbierende Schicht, eine hydrophile Wundkontaktschicht und eine Verteilerschicht, wobei die Wundkontaktschicht ein hydrophiles Polyurethan-Elastomer umfasst, ebenfalls Gegenstand der vorliegenden Erfindung. Insbesondere ist die absorbierende Schicht mit der Wundkontaktschicht verbunden. Eine solche Wundauflage weist besonders vorteilhaft eine Verteilerschicht zwischen der Trägerschicht und der absorbierenden Schicht auf, die insbesondere aus einem hydrophilen Polyurethan-Schaum besteht. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt.

Gemäß einem weiterführenden Gedanken ist auch ein Verfahren zur Herstellung einer mehrschichtigen Wundauflage Gegenstand der vorliegenden Erfindung. Insbesondere ist ein Verfahren zur Herstellung einer oben beschriebenen Wundauflage Gegenstand der vorliegenden Erfindung. Dieses Verfahren umfasst die Verfahrensschritte

a) Bereitstellen eines Polyurethanschaums,
b) Beaufschlagen des Polyurethanschaums mit mindestens 10 Gew.-% Wasser und
c) Laminieren des unter b) hergestellten wasserhaltigen Polyurethanschaums auf eine Wundkontaktschicht.

Insbesondere wird der Polyurethanschaum in diesem Verfahren mit mindestens 10 Gew.-% und höchstens 80 Gew.-% Wasser beaufschlagt, wobei die Beaufschlagung mit Wasser mittels Sprühen oder Tauchen erfolgt.

Hervorzuheben ist an dieser Stelle, dass die hier aufgeführten Merkmale der bevorzugten oder alternativen Ausgestaltungen der Erfindungen nicht auf die einzelnen Bevorzugungen oder Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein.

In den Zeichnungen zeigen:

Figur 1: Eine erste erfindungsgemäße Wundauflage
Figur 2: Eine zweite erfindungsgemäße Wundauflage im Querschnitt
Figur 3 Eine dritte erfindungsgemäße Wundauflage im Querschnitt
Figur 3a Ein Teilausschnitt der dritten erfindungsgemäße Wundauflage im Querschnitt

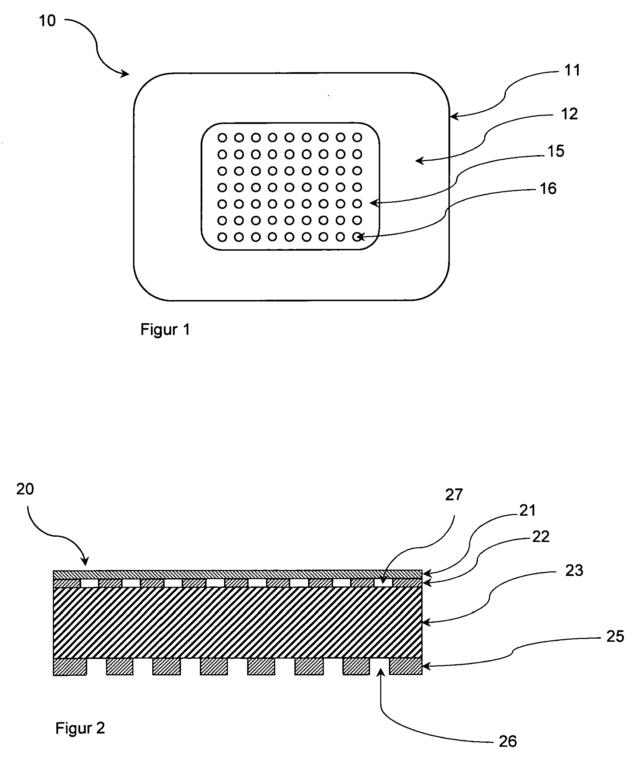

Mit Figur 1 ist eine erste erfindungsgemäße Wundauflage (10) mit Sicht auf die Wundkontaktschicht gezeigt. Die Wundauflage (10) ist als so genannter Inselverband gefertigt und besteht aus einer Trägerschicht (11) aus einem wasserundurchlässigen und wasserdampfdurchlässigen Polyurethanfilm, die vollflächig mit einem Acrylatkleber (12) beschichtet ist. Im Zentrum der Trägerschicht ist mittels des Acrylatklebers (12) eine absorbierende hydrophile Polyurethanschaumschicht aufgebracht (hier nicht dargestellt), auf die ein Polyurethanfilm (15) als Wundkontaktschicht aufgebracht ist. Diese hydrophile Polyurethanschaumschicht umfasst einen Wasseranteil von 40 Gew.-% Wasser. Somit umfassen 100 g eines in diesem Beispiel verwendeten Polyurethanschaums 40 g Wasser und 60 g Polyurethanmatrix. Der Polyurethanfilm (15) ist adhäsiv mit der absorbierenden Polyurethanschaum (hier nicht dargestellt) verbunden. In den Polyurethanfilm sind eine Vielzahl an kreisförmigen Löchern (16) eingebracht, um den Durchfluss von Wundexsudat von der Wunde in die absorbierende Schicht zu ermöglichen. Durch den Polyurethanfilm wird ein Einwachsen von neu gebildeten Zellen in die Poren des Polyurethanschaums verhindert.

Mit Figur 2 ist eine weitere Ausführungsform einer erfindungsgemäßen Wundauflage gezeigt. Die Wundauflage (20) umfasst eine mit einer absorbierenden Schicht (23) kongruente Trägerschicht (21) aus einem wasserundurchlässigen und wasserdampfdurchlässigen Polyurethanschaum, die mittels einer diskontinuierlichen adhäsiven Schicht (22) aus einem Acrylatkleber mit der absorbierenden Schicht (23) verbunden ist. Durch den diskontinuierlichen Auftrag bleiben Bereiche (27) der absorbierenden Schicht und der Trägerschicht unverbunden. Die Wundauflage umfasst eine absorbierende Schicht (23) mit einer Schichtdicke von 4 mm und eine Trägerschicht (11) mit einer Schichtdicke von 1,5 mm. Die absorbierende Schicht (23) wird aus einem offenporigen hydrophilen Polyurethanschaum gebildet, der eine Porengröße von durchschnittlich 220 $\mu$m aufweist. Der Polyurethanschaum umfasst dabei einen Wasseranteil von 70 Gew.-%. Auf die erste Seite des Polyurethanschaums ist ein Polyurethan-Adhäsiv als Wundkontaktschicht (25) aufgebracht. Das Polyurethan-Adhäsiv ist mit einem Flächengewicht von 75 g/ m$^2$ nicht kontinuierlich auf dem Polyurethanschaum aufgebracht, so dass in der Wundkontaktschicht (25) kreisförmige Löcher (26) zum verbesserten Durchtritt von Wundexsudat vorgesehen sind. Der Polyurethanschaum umfasst eine erste Seite mit einer Fläche von 25 cm$^2$, wobei die Löcher (26) insgesamt eine Fläche von 5 cm$^2$ einnehmen.

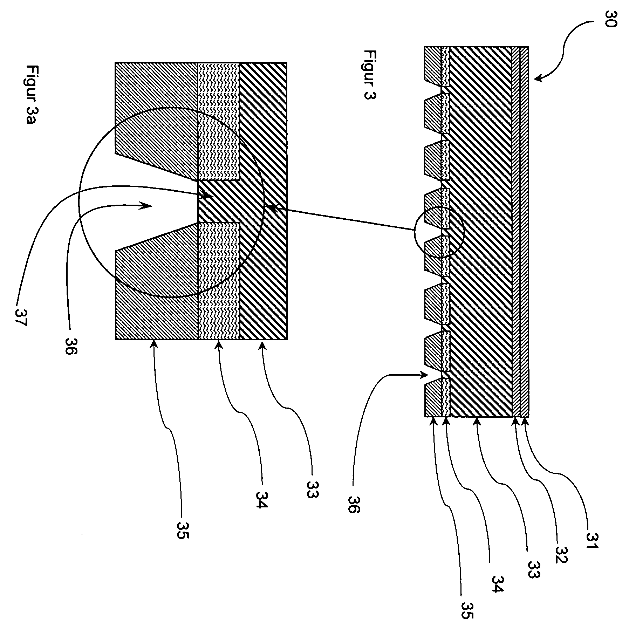

Mit Figur 3 ist eine dritte Ausführung einer erfindungsgemäßen Wundauflage gezeigt. Die Wundauflage (30) umfasst eine Trägerschicht (31) aus einem wasserundurchlässigen und wasserdampfdurchlässigen Polyurethanfilm, eine absorbierende Schicht (33) aus einem offenporigen hydrophilen Polyurethanschaum mit einem Wasseranteil von 52,8 Gew.-% (bezogen auf den Polyurethanschaum) und einer Wundkontaktschicht (35) aus einer wasserhaltigen Hydrogelmatrix mit einem Wasseranteil von ca. 63,5 Gew.-% (bezogen auf das Hydrogel). Die Trägerschicht (31) ist vollflächig mittels einem auf den Polymerfilm aufgetragenem Acrylatkleber (32) auf den hydrophilen Polymerschaum auflaminiert. Auf der im Anwendungsfall zur Wunde weisenden ersten Seite der absorbierenden Schicht ist eine wasserhaltige Hydrogelmatrix (35) aufgebracht, die ein Polyurethan-Polyharnstoff-Copolymer umfasst. Die Hydrogelmatrix ist mit konischen, im Querschnitt (parallel zur Wunde) kreisförmigen Kanälen (36) ausgestattet, so dass ein verbesserter Wundexsudatfluss von der Wunde in den absorbierenden hydrophilen Schaum erfolgen kann (vgl. Figur 3a). Bei der Herstellung der Wundauflage ist die noch viskose Hydrogelmatrix geringfügig in den Polyurethanschaum eingedrungen, so dass zwischen der Hydrogelmatrix und dem hydrophilen Polyurethanschaum eine Übergangsschicht (34) ausgebildet ist, die aus der Hydrogelmatrix und dem hydrophilen Polyurethanschaum besteht. Die Übergangsschicht ihrerseits weist Kanäle (37) auf, die nur mit Polyurethanschaum gefüllt sind und die kongruent zu den Kanälen in der Hydrogelmatrix angeordnet sind.

Ausführungsbeispiel

Die Wundauflage weist den mit Figur 3 beschriebenen Aufbau auf.

A) Herstellung des Hydrogels

Zur Herstellung des Hydrogels werden folgende wässrigen Lösungen und Komponenten (Komponente A, B, C) eingesetzt:

Komponente A

| Propylenglycol USP30 (99,8 %) | Fa. Hedinger Auq. GmbH; Stuttgart, Deutschland | 23,24 Gew.-% |
| Aqua purificata | Wasseraufbereitungsanlage | 75,41 Gew.-% |
| NaCl, reinst, USP | Fa. HedingerAuq. GmbH; Stuttgart, Deutschland | 1,35 Gew.-% |

Zur Herstellung der Komponente A werden die Inhaltsstoffe zusammengegeben und gerührt bis das Salz vollständig gelöst ist. Die Komponente A wird auf 2 °C abgekühlt.

Komponente B

| Jeffamin ED-2003 | Fa. Huntsman; Everberg, Belgien | 52,5 Gew.-% |
| Aqua purificata | Wasseraufbereitungsanlage | 47,5 Gew.-% |

Zur Herstellung der wässrigen Komponente B wird das feste Jeffamin bei 50°C aufgeschmolzen und dem vorgelegten Wasser unter Rühren zugegeben. Die Komponente B wird auf Raumtemperatur abgekühlt.

Komponente C

| Aquapol PI-13000-3 | Fa. Carpenter; Richmond, USA | 100,0 Gel.-% |
|---|---|---|

Die Komponente C wird auf Raumtemperatur gebracht.

Die vorbereiteten Komponenten A, B, und C werden im Verhältnis 75,4:14,0:10,6 miteinander homogen durch homogenisieren mittels eines rotierenden Mischsystems vermischt und in die bereitgestellten Formen möglichst blasenfrei gegossen.

B1) Verwendeter Polyurethanschaums

Es wird ein hydrophiler Polyurethanschaum (Polyurethanschaum MCF.03R; Fa. Corpura, - Etten Leur, Niederlande) verwendet. Der trockene hydrophile Polyurethanschaum weist folgende Charakteristika auf:

a) Dichte: 77,9 - 83,7 kg/ $m^3$ (EN-ISO 845)
b) mittlere Porengröße: $\varnothing$ 208 $\mu$m (Bestimmt mittels Mikroskop)
c) Schichtdicke: 2,7 mm (Dickenmessgerät mit 25 $cm^2$ Teller, 2 $g/cm^2$ Belastung, gemessen nach EN ISO 9073-2)
d) Wasserdampfdurchlässigkeit: MVTR (upright) = 3593 g/ $m^2$/ 24 h (gemessen nach DIN EN 13726-2)
e) Absorption: freie Absorption $A_1$ = 20,5 g/ g (gemessen nach DIN EN 13726-1)
f) Quellvermögen: $\Delta V_0$ = 89,7 %

Das Quellvermögen $\Delta V_0$ eines Polyurethanschaums beschreibt die Volumenänderung, die ein trockener Polyurethanschaum erfährt, nachdem er seine maximale Absorption erreicht hat. Zur Bestimmung des Quellvermögens $\Delta V_0$ werden die räumlichen Abmessungen eines Probenstücks des trockenen Polymerschaums und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 $cm^2$ Teller verwendet, wobei eine Belastung von 2 $g/cm^2$ gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Die Volumenänderung nach Absorption entspricht dem Quellvermögen $\Delta V_0$ des trockenen Polyurethanschaums, wobei alle drei Raumrichtungen beachtet werden.

| | Probe 1 | Probe 2 | Probe 3 | Mittelwert | Änderung / mm (%) |
|---|---|---|---|---|---|
| Länge ($l_0$)/mm | 50,0 | 50,0 | 50,0 | 50,0 | - |
| Breite ($b_0$)/mm | 50,0 | 50,0 | 50,0 | 50,0 | - |
| Höhe ($h_0$)/mm | 2,80 | 2,81 | 2,81 | 2,81 | - |
| Länge ($l_2$)/mm | 60,2 | 61,9 | 61,3 | 61,1 | 11,1 mm (22,2 %) |
| Breite ($b_2$)/mm | 61,7 | 63,5 | 62,8 | 62,7 | 12,7 mm (25,4 %) |
| Höhe ($h_2$)/mm | 3,47 | 3,47 | 3,49 | 3,48 | 0,67 mm (23,8 %) |

$$\Delta V_0 = \frac{V_2 - V_0}{V_0} \bullet 100\% = \frac{(l_2 \bullet b_2 \bullet h_2) - (l_0 \bullet b_o \bullet h_0)}{(l_0 \bullet b_0 \bullet h_0)} \bullet 100\% = 89,7\%$$

wobei gilt: $V_0$ = das Volumen des Probenstücks vor Absorption (gemessen unter Normklimabedingungen (23°C, 50% relative Luftfeuchtigkeit), und
$V_2$ = das Volumen des Probenstücks nach vollständiger Absorption ist

g) Retentionswert: R = 52,8 %

Der Retentionswert R beschreibt die Menge Wasser, die der Polyurethanschaum in seine Polyurethanmatrix maximal einbinden kann, wobei das Wasser, das in die Poren aufgenommen werden könnte, unberücksichtigt bleibt.

Der Retentionswert wird bestimmt, in dem ein Probenstück von 5 cm x 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird dem Probenstück mittels einer Rolle (Gewicht 5000 g, Durchmesser 10 cm, Breite 5 cm) herausgequetscht, in dem die Probe mehrmals zwischen frische Zellstofftücher gelegt und mit der Rolle überrollt wird. Dieser Vorgang wird solange wiederholt, bis keine Wasserabsorption in den Zellstofftüchern erkennbar ist. Zur Bestimmung des Retentionswertes R wird der Wasseranteil $W_{ww}$, der nach dem Absorbieren und dem Ausquetschen in dem Polyurethanschaum enthalten ist gemäß DIN EN 14079 gemessen und wie folgt berechnet.

|  | Probe 1 | Probe 2 | Mittelwert |
|---|---|---|---|
| Gewicht trocken ($W_{tt}$) | 0,57 g | 0,58 g | 0,58 g |
| Gewicht nach Absorption | 11,44 g | 11,75 g | 11,60 g |
| Gewicht nach dem Ausdrücken ($W_{gg}$) | 1,22 g | 1,24 g | 1,23 g |

Die Dicke der vermessenen Probenstücke beträgt 2,80 mm.

$$R = W_{ww} = \frac{W_{gg} - W_{tt}}{W_{gg}} \bullet 100\% = 52,8\,\%$$

(gemessen nach DIN EN 14079)

wobei gilt: $W_{ww}$ = das Gewicht des Wassers, dass nach der Absorption und dem Ausquetschen in der Polyurethanschaum enthalten ist
$W_{tt}$ = das Gewicht des Probenstücks nach dem Trocknen und
$W_{gg}$ = das Gewicht des Probenstücks nach Absorption und nach Ausquetschen

B2) Konditionieren des Polyurethanschaums

Der trockene hydrophile Polyurethanschaum wird auf die Abmessungen 20 x 30 cm zugeschnitten und für 3 Minuten in Wasser getaucht, so dass der Polyurethanschaum seine maximale Absorption erreicht. Der Polyurethanschaum wird aus dem Wasser entfernt und vorsichtig von Hand ausgequetscht. Der Polyurethanschaum wird hiernach mehrmals zwischen trockene Zellstofftücher gelegt und mittels einer Walze ausgequetscht (Liniendruck 10 N/ cm), so lange bis keine Wasserabsorption in den Zellstofftüchern erkennbar ist. Somit kann ausgeschlossen werden, dass kein Wasser in den Poren des Schaums vorhanden ist.
Der wasserhaltige Polyurethanschaum weist folgende Charakteristika auf:

a) Wassergehalt: Der hydrophile Polyurethanschaum hat einen Wasseranteil $W_w$ = 52,8 Gew.-% (gemessen nach DIN EN 14079), der dem Retentionswert R des trockenen Polyurethanschaums entspricht.
b) Absorption: freie Absorption $A_2$ = 16,2 g/ g (gemessen nach DIN EN 13726-1)
c) Quellvermögen: $\Delta V_1$ = 4 %

Das Quellvermögen des wasserhaltigen Polyurethanschaumstoffs wird in analoger Weise zu dem trockenen Polymerschaum bestimmt.

|  | Probe 1 | Probe 2 | Probe 3 | Mittelwert | Änderung 8 mm (%) |
|---|---|---|---|---|---|
| Länge ($l_1$) / mm | 62,8 | 64,3 | 64,15 | 63,75 | - |
| Breite ($b_1$) / mm | 63,4 | 64,45 | 64,3 | 64,05 | - |
| Höbe ($h_1$) / mm | 3,4 | 3,38 | 3,39 | 3,39 | - |
| Länge ($l_2$) / mm | 60,2 | 61,9 | 61,3 | 61,1 | 2,65 mm -4,3%) |
| Breite ($b_2$) / mm | 61,7 | 63,5 | 62,8 | 62,7 | 1,35 mm (2,2%) |

(fortgesetzt)

|  | Probe 1 | Probe 2 | Probe 3 | Mittelwert | Änderung 8 mm (%) |
|---|---|---|---|---|---|
| Höbe ($h_2$) / mm | 3,47 | 3,47 | 3,49 | 3,48 | -0,09 mm (-2,5%) |

$$\Delta V_1 = \frac{V_2 - V_1}{V_1} \bullet 100\% = \frac{(l_2 \bullet b_2 \bullet h_2) - (l_1 \bullet b_1 \bullet h_1)}{(l_1 \bullet b_1 \bullet h_1)} \bullet 100\% = 4\%$$

wobei gilt: $V_1$ = das Volumen des wasserhaltigen Probenstücks und
$V_2$ = das Volumen des Probenstücks nach vollständiger Absorption ist

C) Weitere verwendete Materialien

Als Trägerschicht wird ein 60 $\mu$m dicker, wasserundurchlässiger Polyurethanfilm (Fa. Exopack- Wrexham, United Kingdom) verwendet. Dieser Film ist mit einer Schichtdicke von 30 $\mu$m mit einem Pressure Sensitive Adhesive auf Acrylatbasis beschichtet. Der Film weist eine Wasserdampfdurchlässigkeit MVTR (upright) von 1100 g/ m$^2$/ 24 h (DIN EN 13726-1) auf.

D) Herstellung der Wundauflagen

Die Wundauflagen (Muster) werden von Hand nach folgendem Ablauf gefertigt.

1. Der Polyurethanschaum wird gemäß B) vorkonditioniert und bereitgestellt.
2. Zur Herstellung einer Hydrogelmatrix mit Kanälen wird eine PTFE-Form mit Noppenstruktur bereitgestellt. Die Noppen der Noppenstruktur sind kegelförmig und haben einen mittleren Durchmesser von 1,38 mm (Sockel 1,56 mm, Spitze 1,2 mm). Die Noppen haben eine Höhe von 1,35 mm und stehen in einem Rechteckraster im Abstand von 5 mm.
3. Das Hydrogel wird gemäß A) hergestellt und bereitgestellt, wobei das Hydrogel nach dem durchmischen und homogenisieren sofort weiterverarbeitet werden muss. Hierzu wird das Hydrogel zur Ausbildung der Hydrogelmatrix in die bereitgestellten Formen möglichst blasenfrei gegossen.
4. Das Gel wird mit einem PTFE-Rakel so verteilt, dass die Gelschicht die Höhe der Noppen (1,35 mm) aufweist. Überschüssiges Gel wird aus der Form entfernt.
5. Nach ca. 3 Minuten wird der vorkonditionierte Polyurethanschaum auf die Geloberfläche gelegt. Der Schaum wird mit einem Druck von 200 N/ m$^2$ angedrückt und gehalten.
6. Nach weiteren ca. 7 Minuten hat sich das Gel mit dem Schaum verbunden, so dass das Laminat aus wasserhaltigem Polyurethanschaum und wasserhaltigen Hydrogelmatrix aus der Form entfernt werden kann. Es wurde eine 0,3 mm dicke Übergangsschicht gebildet, die aus wasserhaltiger Hydrogelmatrix und wasserhaltigem Polyurethanschaum besteht.
7. Das Laminat wird mit der Hydrogelseite auf die vorbereitete Release-Folie gelegt (die silikonisierte Seite ist zum Gel gerichtet), so dass die zur Wunde gerichtete Seite geschützt ist.
8. Der Verbund wird auf der Schaumseite mit einem selbstklebenden Polyurethanfilm (vgl. D) abgedeckt, wobei der Polyurethanfilm mit einem Druck von 200 N/ m$^2$ fest angedrückt wird.
9. Aus dem mehrlagigen Materialverbund werden Wundauflagen mit einer Kantenlänge von 10 x 10 cm ausgestanzt.

Die somit hergestellte Wundauflage weist den mit Figur 3 beschriebenen Aufbau auf, wobei mit Figur 3 kein Releaseliner gezeigt ist. Die Wundauflage besteht somit aus einem Laminat aus einer flexiblen, wasserhaltigen Hydrogelmatrix als Wundkontaktschicht, die 63,5 Gew.-% Wasser (bezogen auf die Hydrogelmatrix) enthält und einer absorbierende Schicht aus einem offenporigen hydrophilen Polyurethanschaum mit einem Wasseranteil von 52,8 Gew.-% (bezogen auf den Polyurethanschaum).
Des Weiteren weist die Wundauflage folgende Charakteristika auf:

a) Flächengewicht: 1550 g/ m$^2$ (gemessen nach DIN EN 29073-1)

b) Absorption: freie Absorption $A_1$ = 56 g/ 100 cm$^2$ (gemessen nach DIN EN 13726-1)

c) Wassergehalt insgesamt: $W_w = \dfrac{W_g - W_t}{W_g} \bullet 100\% = 58{,}8\%$

d) Quellvermögen: $\Delta V = 10\ \%$ (bestimmt gemäß oben beschriebener Methode)

Die vorliegende Wundauflage weist somit einen hohen Wasseranteil und eine hohe Absorption und eine geringe Quellung auf. Die Wundauflage ist somit bestens geeignet um in den Wundheilungsphasen 2 und 3 (Granulationsphase und Epithelisierungsphase) eingesetzt zu werden.

**Patentansprüche**

1. Mehrschichtige Wundauflage umfassend

    a) eine erste Schicht als Wundkontaktschicht mit einer ersten Seite und einer zweiten Seite, und
    b) mindestens einer zweiten Schicht als absorbierende Schicht mit einer ersten Seite und einer zweiten Seite, die einen hydrophilen Polyurethanschaum umfasst,

worin der Polyurethanschaum einen Wasseranteil von mindestens 10 Gew.-% Wasser umfasst, **dadurch gekennzeichnet, dass** der Polyurethanschaum höchstens 80 Gew.-% Wasser umfasst und eine freie Absorption $A_2$ von mindestens 10 g/g aufweist, wobei die freie Absorption gemäß DIN-EN 13726-1 (2002) bestimmt wird.

2. Wundauflage nach Anspruch 1, **dadurch gekennzeichnet, dass** das Wasser homogen in dem Polyurethanschaum verteilt ist.

3. Wundauflage nach mindestens einem der vorhergehenden Ansprüche, **dadurch gekennzeichnet, dass** der Polyurethanschaum eine Porengröße von 100 bis 500 $\mu$m aufweist.

4. Wundauflage nach mindestens einem der vorhergehenden Ansprüche, **dadurch gekennzeichnet, dass** der Polyurethanschaum ein Quellvermögen: $\Delta V_1$ von höchstens 80 % aufweist.

5. Wundauflage nach mindestens einem der vorhergehenden Ansprüche, **dadurch gekennzeichnet, dass** die Wundkontaktschicht eine Vielzahl an Löchern, Kanälen oder Öffnungen aufweist.

6. Wundauflage nach mindestens einem der vorhergehenden Ansprüche, **dadurch gekennzeichnet, dass** die Wundkontaktschicht eine Hydrogelmatrix, einen Polymerfilm, eine Hydrokolloidmatrix, ein Polymernetz, ein Nonwoven oder ein Adhäsiv umfasst.

7. Wundauflage nach Anspruch 6, **dadurch gekennzeichnet, dass** die Hydrogelmatrix eine wasserhaltige Hydrogelmatrix mit einem Wassergehalt von mindestens 20 Gew.-% ist.

8. Wundauflage nach Anspruch 6 oder 7, **dadurch gekennzeichnet, dass** die Hydrogelmatrix ein Polyurethan-Polyharnstoff-Copolymer umfasst.

9. Wundauflage nach Anspruch 6, 7 oder 8, **dadurch gekennzeichnet, dass** die Hydrogelmatrix zum Durchtritt von Flüssigkeiten Kanäle von der ersten zur zweiten Seite aufweist.

10. Wundauflage nach mindestens einem der vorhergehenden Ansprüche, **dadurch gekennzeichnet, dass** die Wundauflage als Trägerschicht weiterhin einen wasserundurchlässigen und wasserdampfdurchlässigen Polymerfilm oder einen wasserundurchlässigen und wasserdampfdurchlässigen Polymerschaum umfasst.

11. Verfahren zur Herstellung einer mehrschichtigen Wundauflage, die als absorbierende Schicht einen Polyurethanschaum umfasst, insbesondere gemäß einem der vorangehenden Ansprüche, wobei das Verfahren die Verfahrensschritte umfasst:

a) Bereitstellen eines Polyurethanschaums,

b) Beaufschlagen des Polyurethanschaums mit mindestens 10 Gew.-% Wasser und

c) Laminieren des unter b) hergestellten wasserhaltigen Polyurethanschaums auf eine Wundkontaktschicht.

**12.** Verfahren zur Herstellung einer mehrschichtigen Wundauflage gemäß Anspruch 11, wobei der Polymerschaum mit mindestens 10 Gew.-% und höchstens 80 Gew.-% Wasser beaufschlagt wird, und wobei das Wasser mittels Sprühen oder Tauchen erfolgt.

**Claims**

**1.** Multilayered wound dressing comprising

a) a first layer as a wound contact layer having a first side and a second side, and

b) at least one second layer as an absorbent layer having a first side and a second side and comprising a hydrophilic polyurethane foam,

wherein the polyurethane foam comprises a water fraction of at least 10% by weight of water, **characterized in that** the polyurethane foam comprises at most 80% by weight of water and has a free absorbency $A_2$ of at least 10 g/g, the free absorbency being determined as per DIN-EN 13726-1 (2002).

**2.** Wound dressing according to Claim 1, **characterized in that** the water is homogeneously distributed in the polyurethane foam.

**3.** Wound dressing according to at least one of the preceding claims, **characterized in that** the polyurethane foam has a pore size in the range from 100 to 500 $\mu$m.

**4.** Wound dressing according to at least one of the preceding claims, **characterized in that** the polyurethane foam has a swell capacity $\Delta V_1$ of at most 80%.

**5.** Wound dressing according to at least one of the preceding claims, **characterized in that** the wound contact layer has a multiplicity of holes, channels or openings.

**6.** Wound dressing according to at least one of the preceding claims, **characterized in that** the wound contact layer comprises a hydrogel matrix, a polymer film, a hydrocolloid matrix, a polymer net, a nonwoven or an adhesive.

**7.** Wound dressing according to Claim 6, **characterized in that** the hydrogel matrix is a water-containing hydrogel matrix having a water content of at least 20% by weight.

**8.** Wound dressing according to Claim 6 or 7, **characterized in that** the hydrogel matrix comprises a polyurethane-polyurea copolymer.

**9.** Wound dressing according to Claim 6, 7 or 8, **characterized in that** the hydrogel matrix has channels from the first to the second side to allow liquids to pass through the hydrogel matrix.

**10.** Wound dressing according to at least one of the preceding claims, **characterized in that** the wound dressing further comprises a water impermeable and moisture vapor permeable polymer film or a water impermeable and moisture vapor permeable polymer foam as a backing layer.

**11.** Process for producing a multilayered wound dressing comprising a polyurethane foam as an absorbent layer, more particularly according to any one of the preceding claims, the process comprising the steps of:

a) providing a polyurethane foam,

b) applying at least 10% by weight of water to the polyurethane foam, and

c) laminating the water-containing polyurethane foam produced under b) onto a wound contact layer.

**12.** Process for producing a multilayered wound dressing according to Claim 11, wherein the amount of water applied to the polymer foam amounts to at least 10% by weight and at most 80% by weight and the water is applied by spraying or dipping.

**Revendications**

1. Pansement multicouche, comprenant .

   a) une première couche en tant que couche de contact avec la lésion, comprenant un premier côté et un second côté, et
   b) au moins une seconde couche en tant que couche absorbante, comprenant un premier côté et un second côté, qui comprend une mousse de polyuréthane hydrophile,

   la mousse de polyuréthane comprenant une proportion d'eau d'au moins 10 % en poids d'eau,
   **caractérisé en ce que** la mousse de polyuréthane comprend au plus 80 % en poids d'eau et présente une absorption libre $A_2$ d'au moins 10 g/g, l'absorption libre étant déterminée selon DIN-EN 13726-1 (2002).

2. Pansement selon la revendication 1, **caractérisé en ce que** l'eau est distribuée de manière homogène dans la mousse de polyuréthane.

3. Pansement selon au moins l'une quelconque des revendications précédentes, **caractérisé en ce que** la mousse de polyuréthane présente une taille de pore de 100 à 500 $\mu$m.

4. Pansement selon au moins l'une quelconque des revendications précédentes, **caractérisé en ce que** la mousse de polyuréthane présente une capacité de gonflement $\Delta V_1$ d'au plus 80 %.

5. Pansement selon au moins l'une quelconque des revendications précédentes, **caractérisé en ce que** la couche de contact avec la lésion comprend une pluralité de trous, de canaux ou d'ouvertures.

6. Pansement selon au moins l'une quelconque des revendications précédentes, **caractérisé en ce que** la couche de contact avec la lésion comprend une matrice d'hydrogel, un film polymère, une matrice d'hydrocolloïde, un réseau polymère, un non-tissé ou un adhésif.

7. Pansement selon la revendication 6, **caractérisé en ce que** la matrice d'hydrogel est une matrice d'hydrogel contenant de l'eau ayant une teneur en eau d'au moins 20 % en poids.

8. Pansement selon la revendication 6 ou 7, **caractérisé en ce que** la matrice d'hydrogel comprend un copolymère de polyuréthane-polyurée.

9. Pansement selon la revendication 6, 7 ou 8, **caractérisé en ce que** la matrice d'hydrogel comprend des canaux du premier vers le second côté pour le passage de liquides.

10. Pansement selon au moins l'une quelconque des revendications précédentes, **caractérisé en ce que** le pansement comprend en tant que couche support en outre un film polymère imperméable à l'eau et perméable à la vapeur d'eau ou une mousse polymère imperméable à l'eau et perméable à la vapeur d'eau.

11. Procédé de fabrication d'un pansement multicouche, qui comprend en tant que couche absorbante une mousse de polyuréthane, notamment selon l'une quelconque des revendications précédentes, le procédé comprenant les étapes de procédé suivantes :

    a) la préparation d'une mousse de polyuréthane,
    b) le chargement de la mousse de polyuréthane avec au moins 10 % en poids d'eau, et
    c) la stratification de la mousse de polyuréthane contenant de l'eau fabriquée en b) sur une couche de contact avec la lésion.

12. Procédé de fabrication d'un pansement multicouche selon la revendication 11, dans lequel la mousse polymère est chargée avec au moins 10 % en poids et au plus 80 % en poids d'eau, et dans lequel l'hydratation a lieu par pulvérisation ou immersion.

Figur 1

Figur 2

Figur 3

Figur 3a

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 457977 B1
- EP 486522 B1
- EP 541390 B1
- EP 541391 B1
- EP 570430 B1
- EP 665856 B1
- EP 691113 B1
- EP 693913 B1
- EP 1082146 B1
- EP 855921 B1

- EP 1156838 B1
- WO 2428581 A
- WO 0238097 A1
- WO 0245761 A1
- WO 03011352 A1
- WO 03086255 A1
- WO 2004052415 A1
- EP 1658865 A1
- US 20040153040 A1
- EP 1007597 B1